# EUROPEAN PATENT SPECIFICATION

(11) **EP 3 003 477 B1**
(45) Date of publication and mention of the grant of the patent: **07.03.2018**
(21) Application number: 14729053.0
(22) Date of filing: 26.05.2014
(51) Int. Cl.: A61N 1/39

(54) **DUAL BATTERY FAST CHARGING DEFIBRILLATOR**
SCHNELL LADENDER DEFIBRILLATOR MIT ZWEI BATTERIEN
DEFIBRILLATEUR A CHARGE RAPIDE A DEUX BATTERIES

(30) Priority: 28.05.2013 US 201361827788 P
(43) Date of publication of application: 13.04.2016
(73) Proprietor: Koninklijke Philips N.V., 5656 AE Eindhoven (NL)
(72) Inventor: DELISLE, Norman Maurice, NL-5656 AE Eindhoven (NL); WUTHRICH, Scott Alan, NL-5656 AE Eindhoven (NL); KOZIN, Simon Edward, NL-5656AE Eindhoven (NL)
(74) Representative: Steffen, Thomas
(86) International application number: PCT/IB2014/061707
(87) International publication number: WO 2014/191889

(56) References cited:
- WO-A1-2009/018535
- US-A- 4 323 075
- US-A1- 2003 193 245
- US-A1- 2010 114 235

## Description

### BACKGROUND:

### Technical Field

This disclosure relates to medical instruments and more particularly to a fast charging defibrillator.

### Description of the Related Art

An external defibrillator needs to be highly portable so that it can be quickly carried to a patient who has sudden cardiac arrest to deliver a life-saving shock. The defibrillator typically operates on battery power. To deliver a shock, the defibrillator charges a capacitor to a high voltage - typically to over 2000 volts. Due to limitations on the discharge rate of batteries, it may require from 5 to 8 seconds or more to charge the capacitor. After sudden cardiac arrest, the chances of survival drop by 10% to 15% every minute, and several shocks may be needed to resuscitate the patient.

Discharge rate of batteries is specified as a fraction of capacity. Thus, a standard Lithium-ion battery cell designed for 2200 milli-amp-hour capacity and a maximum discharge rate of one times capacity (1C), can supply a peak discharge current of 2.2 amps. Lithium ion cells optimized for capacity can handle peak currents of 2C or 3C for short periods. However for safety, the maximum current output is usually limited to 1C or 1.5C.

WO 2009/018535 describes an implantable defibrillator with two batteries along with multiple switches to enable a plurality of battery and power circuit configurations. The power circuit configurations may be changed in response to changes in battery status or device operation.

### SUMMARY

The present invention is set out in the appended claims.

In accordance with the present principles, a defibrillator includes a first battery configured to provide a discharge rate to a capacitor suitable for generating a defibrillator shock. A power source has a larger capacity than the first battery. A power control module is coupled to the first battery and the power source and is configured to selectively enable connections between the first battery and the power source and from each of the first battery and the power source to the capacitor to reduce charge time.

Another defibrillator includes a first battery configured to provide a discharge rate to a capacitor suitable for generating a defibrillator shock. A second battery has a larger capacity than the first battery. A power control module is coupled between the first battery and the second battery and is configured to selectively enable connections between the first battery and the second battery and from each of the first battery and the second battery to the capacitor to reduce charge time and increase a recovery rate after a discharge. A delivery circuit is coupled to the power control module and the capacitor and configured to enable discharge of the capacitor to a shock delivery device, the delivery circuit providing feedback to the power control module for a state of the capacitor.

A method for charging a defibrillator includes providing a first battery configured to provide a discharge rate to a capacitor suitable for generating a defibrillator shock, a power source having a larger capacity than the first battery, and a power control module coupled to the first battery and the power source and configured to selectively enable connections between the first battery and the power source and from each of the first battery and the power source; and charging the capacitor in accordance with the power control module which selectively enables the connections in accordance with feedback from at least one of the first battery and the power source to reduce charge time of the capacitor.

These and other objects, features and advantages of the present disclosure will become apparent from the following detailed description of illustrative embodiments thereof, which is to be read in connection with the accompanying drawings.

### BRIEF DESCRIPTION OF DRAWINGS

This disclosure will present in detail the following description of preferred embodiments with reference to the following figures wherein:
FIG. 1 is a block/flow diagram showing a dual source defibrillator in accordance with one embodiment;
FIG. 2 is a block/flow diagram showing power control circuit or module for the dual source defibrillator in accordance with one embodiment; and
FIG. 3 is a flow diagram showing a method for charging a defibrillator in accordance with an illustrative embodiment.

### DETAILED DESCRIPTION OF EMBODIMENTS

In accordance with the present principles, a high discharge rate battery technology is employed to reduce defibrillator charge times by a factor of 8 or more. This results in a defibrillator that can charge its capacitor in less than one second. Because high discharge rate batteries have lower overall charge capacity, this high discharge rate battery can be complemented with a standard discharge rate battery to provide long battery operation times for the defibrillator.

The high-discharge rate battery achieves extremely fast capacitor charge times. There are several exemplary embodiments of a defibrillator that combine a high discharge rate battery with a high capacity battery. For pre-hospital usage, the high discharge rate battery can be used to charge the capacitor while a second battery with high capacity cells is used to power other operations and provide longer run times. For hospital usage where needed runtimes are shorter, the high discharge rate battery can be used to both operate the device and charge the capacitor. A high discharge rate battery is used to power the defibrillator instead of the high capacity battery to significantly reduce the time needed to charge the defibrillator capacitor so that a shock can be delivered to a patient more rapidly. Battery capacity varies with the discharge rate; therefore, a high discharge rate battery has a lower capacity than a standard discharge rate battery. To compensate for the reduced capacity of the high discharge rate battery, a second battery with high capacity can be used to provide extended runtimes when not charging the capacitor.

By way of reference, a 12 battery pack with a 4 serial/3 parallel arrangement of standard 2200 milli-amp-hour Lithium ion cells optimized for capacity can safely discharge at a maximum current of about 11 amps for a few seconds. This is sufficient power to charge a defibrillator capacitor in about 4 seconds for a 200J shock or about 7 seconds for a 360J shock. This type of battery pack will provide approximately 6.6 amp-hours capacity.

Lithium ion cells optimized for power can deliver pulsed currents of 30C to 40C. Thus, even a much smaller battery pack with just 4 serial Lithium ion cells optimized for discharge rate can discharge at a maximum current of about 40 amps. This is sufficient power to charge a defibrillator capacitor for a 200J shock in about a second. The drawback is that this type of battery pack will only provide approximately 1.6 amp-hours capacity. The present principles address these issues.

It also should be understood that the present invention will be described in terms of medical instruments; however, the teachings of the present invention are much broader and are applicable to any battery operated device that needs to draw higher current for short durations. In some embodiments, the present principles are employed in defibrillators used in hospitals or emergency vehicles, in homes, public places, etc. The elements depicted in the FIGS. may be implemented in various combinations of hardware and software and provide functions which may be combined in a single element or multiple elements.

The functions of the various elements shown in the FIGS. can be provided through the use of dedicated hardware as well as hardware capable of executing software in association with appropriate software. When provided by a processor or controller, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor ("DSP") hardware, read-only memory ("ROM") for storing software, random access memory ("RAM"), non-volatile storage, etc.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (i.e., any elements developed that perform the same function, regardless of structure). Thus, for example, it will be appreciated by those skilled in the art that the block diagrams presented herein represent conceptual views of illustrative system components and/or circuitry embodying the principles of the invention. Similarly, it will be appreciated that any flow charts, flow diagrams and the like represent various processes which may be substantially represented in computer readable storage media and so executed by a computer or processor, whether or not such computer or processor is explicitly shown.

Furthermore, embodiments of the present invention can take the form of or include a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Examples of a computer-readable medium include a semiconductor or solid state memory, magnetic tape, a removable computer diskette, a random access memory (RAM), a read-only memory (ROM), a rigid magnetic disk and an optical disk. Current examples of optical disks include compact disk - read only memory (CD-ROM), compact disk - read/write (CD-R/W), Blu-Ray™ and DVD

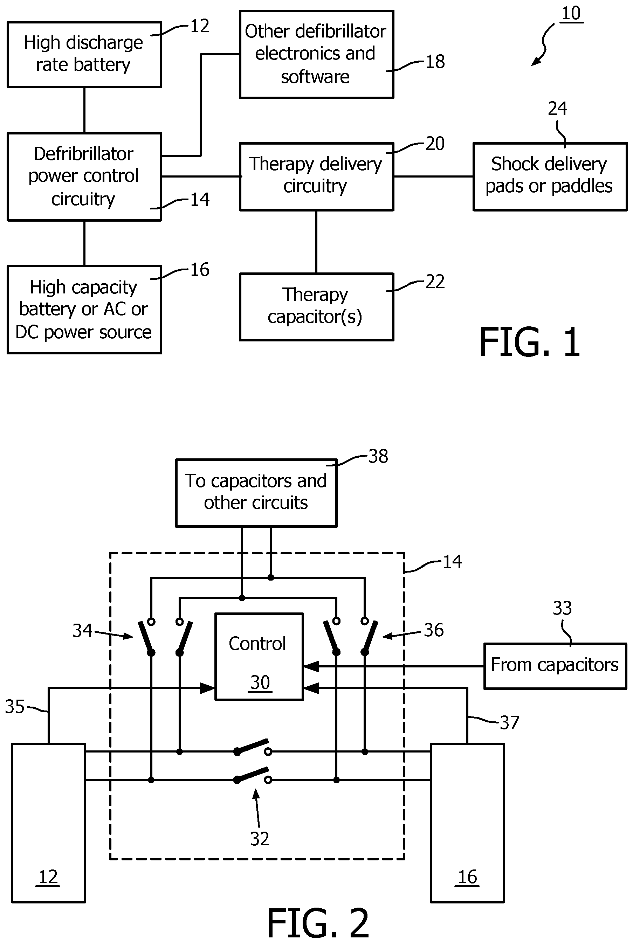

Referring now to the drawings in which like numerals represent the same or similar elements and initially to FIG. 1, a block/flow diagram shows a defibrillator 10 in accordance with one embodiment. Defibrillator 10 includes a high discharge rate battery 12 and a high capacity power source 16. The selection of the type and usage of the energy sources (12, 16) is determined and controlled by defibrillator power control circuitry 14. Therapy delivery circuitry 20 controls the amount of energy and shapes of pulses delivered to shock delivery devices 24, which may include pads or paddles. Therapy delivery circuitry 20 provides access to therapy capacitors 22 to enable charging and discharging thereof. Defibrillator 10 may include other electronics or software programs 18. The electronics 18 may include other features, such as clocks, timers, power measurement devices, a light, a display, etc.

When the defibrillator 10 needs to deliver a shock, the therapy delivery circuitry 20 charges the therapy capacitor(s) 22 by routing current from the high discharge rate battery 12. When a user triggers the release of energy, the energy stored in therapy capacitor(s) 22 is discharged via pads or paddles 24 placed on a patient's chest. The power control circuitry 14 routes power from the available power sources, battery 12 and/or high capacity power source 16. The power for the defibrillator 10 is supplied by the high discharge rate battery 12. This battery 12 is charged by a second power source 16, which may include a high capacity battery (or an AC or DC battery charger).

The high discharge rate battery 12 is used to power the defibrillator 10 and charge the capacitor(s) 22. The capacitor(s) 22 is charged using the higher current output of the battery 12. The capacitor(s) 22 may also charged from the high capacity battery 16 (or an AC or DC charger).

Referring to FIG. 2, the power control circuitry 14 may include control software or circuitry controller 30 configured to determine the available capacity of each of the batteries 12 and 16 and may regulate access to battery terminals depending on the power requirements of the capacitors 22 (FIG. 1). The batteries or energy sources 12, 16 also provide data interfaces 35, 37, respectively, so that their current charge levels and other status can be read by the controller 30. The capacitors 22 and/or batteries 12, 16 provide feedback 33 to the power control circuitry 14 to permit the power control circuitry 14 to determine how quickly the capacitors 22 are charging or recharging or to determine the capabilities of the batteries 12, 16 for future use.

The data sent to the control circuitry or module 30 may include data bits or other signals configured to convey the amount of capacity/amount of charge/status of the batteries of power sources. The data may also include a charge rate from the capacitors 33. The input or feedback information may be employed to make a decision based upon a lookup table or other data structure stored in the control module 30. The data may be indexed to the data structure to determine the connection to be enabled/disabled or to otherwise control the power distribution between power sources. Other decision criteria may be employed as well including but not limited to programmed actions under given conditions, historic data, artificial intelligence, etc. For example, based on the charging rate or stored energy in the batteries 12, 16, especially after a shock has been deployed, the controller 30 selects whether to recharge the battery 12 from battery 16, recharge the capacitors directly from the battery 12, recharge the capacitors directly from the battery 16, or open up both batteries 12 and 16 to recharge the capacitors 22. The use of both sources becomes difficult when the sources include significantly different discharge rates. Charge regulators and other circuitry may be employed to condition the signal. The available charge of the batteries is a more important criterion. For example, if the high discharge battery has low charge, it might not be able to fully charge the capacitors. Then, the other power source would be selected and employed.

In one embodiment, the controller 30 selectively activates one or more switches 32, 34 and 36 to enable power output 38 from one, the other or both batteries 12 and 16. In one embodiment, the battery 16 may include a different DC source or even an AC source. In another embodiment, there may be more than two batteries and each battery may be selectively enabled using the controller 30.

In useful embodiments, the batteries 12 and 16 may include one or more of the following. A standard Lithium-ion battery cell, such as a CGR18650CG has a capacity of 2,200 mAh and a maximum discharge rate of 1C. Generally these cells are configured in series to realize the needed voltage (e.g., 4S provides a nominal voltage of 14.4V) and in parallel to increase the total discharge current (e.g., 3P provides a 3C maximum discharge rate). A 12 cell (4S3P) battery pack provides a capacity of 6.6 Amp-hours, with a maximum discharge of 2 Amps (derating the maximum discharge rate to improve reliability). High discharge rate battery cells are designed for applications such as power tools and remote-controlled model cars and planes where higher currents are needed to drive electric motors. A high discharge rate Lithium-ion cell, such as a APR18650M1, has a capacity of 1,100 mAh. A 4 cell (4S1P) battery pack provides a capacity of 1.1 Amp-hours and can reliably provide discharges of 30 Amps.

Different combinations of the elements presented in FIG. 1 can be employed to optimize a defibrillator / monitor for its intended use. Exemplary embodiments may include the following. Battery 12 may include a single four cell high discharge rate battery (4S, 1P) for charging the capacitor 22 plus one or more eight or twelve cell high capacity batteries (4S and either 2P or 3P) for battery 16 to power other device functions. An eight or twelve cell high discharge rate battery (4S and either 2P or 3P) (battery 12) may be employed for powering all device functions plus an AC or DC battery charger for power source 16. A single four, eight or twelve cell high discharge rate battery (4S and either 1P or 2P or 3P) (battery 12) may be employed to power the device plus one or more ten cell batteries (5S, 2P) (battery 16) to charge the high discharge rate batteries (12).

In accordance with the present principles, the defibrillator 10 provides pulsed currents of 30C to 40C with just four serial lithium ion cells optimized for discharge rate and can discharge at a maximum current of about 30 amps using the battery 12. Control of the pulses may be provided by the delivery circuitry 20 or other circuits in the defibrillator 10. This is sufficient power to charge a defibrillator capacitor for a 200J shock in about a second. Since the defibrillator 10 includes a second power source 16 that may include a higher capacity battery. This battery 16 can provide over 6.0 amp-hours capacity and is sufficient to charge the first battery 12 and/or the capacitors 22.

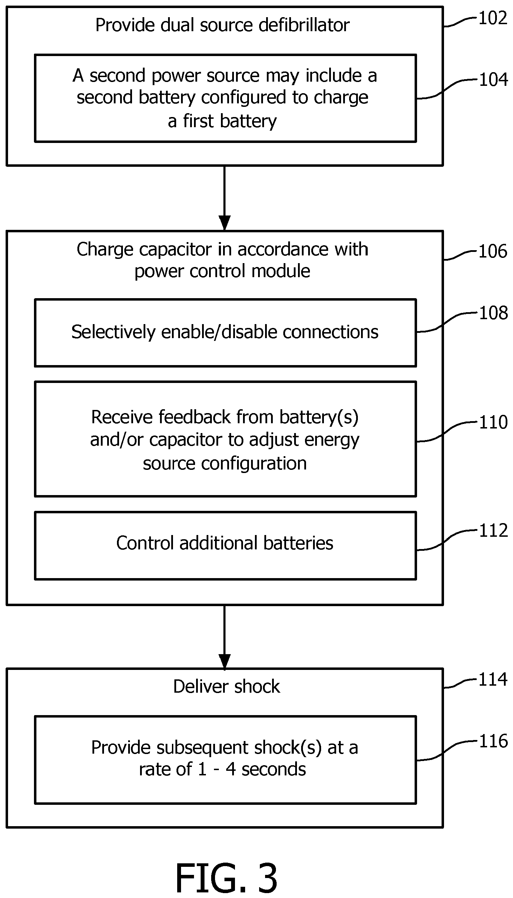

Referring to FIG. 3, a block/flow diagram shows a method for charging a defibrillator in accordance with the present principles. In block 102, a dual source defibrillator is provided having a first battery configured to provide a discharge rate to a capacitor suitable for generating a defibrillator shock, a power source having a larger capacity than the first battery, and a power control module coupled to the first battery and the power source. The power control module is configured to selectively enable connections between the first battery and the power source and from each of the first battery and the power source to a capacitor. In block 104, the power source may include a second battery, a direct current source or an alternating current source configured to charge the first battery in accordance with the power control module.

In block 106, the capacitor is charged in accordance with the power control module to reduce charge time of the capacitor and/or increase a recovery rate after a discharge of the capacitor. In block 108, connections between the first battery and the power source and between each of the first battery and the power source to the capacitor are selectively enabled or disabled to improve a charging rate of the capacitor. In block 110, feedback is received from the capacitor and /or batteries at the power control module to adjust (e.g., optimize) a configuration of energy sources (batteries, etc.) to increase a charge rate of the capacitor. In block 112, one or more additional batteries are controlled by the power control module. Optimization decisions may be made by the power control module or may be preprogrammed in memory and made in accordance with current conditions.

In block 114, a shock is delivered by discharging the capacitor through a shock delivery device. Subsequent shocks may be administered at a rate of about every 1-4 seconds in block 116.

## Claims

1. A defibrillator, comprising:
a first battery (12) configured to provide a discharge rate to a capacitor (22) suitable for generating a defibrillator shock;
a power source (16); and
a power control module (14, 30) coupled to the first battery (12) and the power source (16) and configured to selectively enable connections between the first battery (12) and the power source (16) and from each of the first battery (12) and the power source (16) to the capacitor (22), said defibrillator being **characterized in that**
the power source (16) has a larger capacity than the first battery (12), **in that**
the power control module (14, 30) is configured to selectively enable said connections based on feedback from the capacitor (22), from the first battery (12) and from the power source (16) to adjust a configuration of energy sources to increase a charge rate of the capacitor (22).

2. The defibrillator as recited in claim 1, wherein the power source (16) includes a second battery.

3. The defibrillator as recited in claim 1, wherein the power source (16) includes one of a direct current source and an alternating current source.

4. The defibrillator as recited in claim 1, wherein the power control module (14, 30) includes a controller (30) configured to selectively enable or disable connections (32, 34, 36) between the first battery (14) and the power source (16) and between each of the first battery (14) and the power source (14) to the capacitor (22).

5. The defibrillator as recited in claim 1, wherein the power source (16) is configured to charge the first battery (12).

6. The defibrillator as recited in claim 1, further comprising one or more additional batteries controlled by the power control module.

7. A defibrillator, comprising:
a first battery (12) configured to provide a discharge rate to a capacitor suitable for generating a defibrillator shock;
a second battery (16); a power control module (14) coupled between the first battery and the second battery and configured to selectively enable connections between the first battery and the second battery and from each of the first battery and the second battery to the capacitor; and
a delivery circuit (20) coupled to the power control module and the capacitor and configured to enable discharge of the capacitor to a shock delivery device, the delivery circuit providing feedback to the power control module for a state of the capacitor, **characterized in that**
the second battery (16) has a larger capacity than the first battery (14), **in that**
the power control module (14) is configured to selectively enable said connections based on feedback received from the first and second batteries through the delivery circuit (20) and on feedback received from the capacitor (22) through the delivery circuit (20) to adjust a configuration of energy sources to increase a charge rate of the capacitor.

8. The defibrillator as recited in claim 7, wherein the power control module (14) includes a controller (30) configured to selectively enable or disable connections (32, 34, 36) between the first battery and the second battery and between each of the first battery and the second battery to the capacitor.

9. The defibrillator as recited in claim 7, wherein the second battery (16) is configured to charge the first battery (12).

10. The defibrillator as recited in claim 9, further comprising one or more additional batteries controlled by the power control module.

11. A method for charging a defibrillator, comprising:
providing (120) a first battery configured to provide a discharge rate to a capacitor suitable for generating a defibrillator shock, a power source, and a power control module coupled to the first battery and the power source and configured to selectively enable connections between the first battery and the power source and from each of the first battery and the power source to the capacitor; and
charging (106) the capacitor in accordance with the power control module which selectively enables the connections in accordance with feedback, **characterized in that**
the power source (16) has a larger capacity than the first battery (14), **in that** the method comprises:
receiving (110) feedback from the capacitor, the first battery and the power source at the power control module (14) to adjust a configuration of energy sources to increase a charge rate of the capacitor.

12. The method as recited in claim 11, wherein the power source includes a second battery configured to charge the first battery in accordance with the power control module.

13. The method as recited in claim 11, wherein the power source includes one of a direct current source and an alternating current source.

14. The method as recited in claim 11, further comprising selectively enabling or disabling (108) connections between the first battery and the power source and between each of the first battery and the power source to the capacitor to improve a charging rate of the capacitor.

15. The method as recited in claim 11, further comprising at least one of
(i) controlling (112) one or more additional batteries by the power control module, or
(ii) delivering (114) a shock by discharging the capacitor through a shock delivery device.

## Patentansprüche

1. Defibrillator, umfassend:
eine erste Batterie (12), die konfiguriert ist, um eine Entladungsrate für einen Kondensator (22) bereitzustellen, die für das Erzeugen eines Defibrillierschocks geeignet ist;
eine Stromversorgungsquelle (16); und
ein Leistungssteuerungsmodul (14, 30), das mit der ersten Batterie (12) und der Stromversorgungsquelle (16) gekoppelt ist und konfiguriert ist, um selektiv Verbindungen zwischen der ersten Batterie (12) und der Stromversorgungsquelle (16) und von sowohl der ersten Batterie (12) als auch von der Stromversorgungsquelle (16) zum Kondensator (22) herzustellen, wobei der genannte Defibrillator
**dadurch gekennzeichnet ist, dass**
die Stromversorgungsquelle (16) eine größere Kapazität hat als die erste Batterie (12), dass das Leistungssteuerungsmodul (14, 30) konfiguriert ist, um basierend auf Rückmeldungen von dem Kondensator (22), von der ersten Batterie (12) und von der Stromversorgungsquelle (16) selektiv Verbindungen herzustellen, um eine Konfiguration von Energiequellen anzupassen, um eine Aufladerate des Kondensators (22) zu erhöhen.

2. Defibrillator nach Anspruch 1, wobei die Stromversorgungsquelle (16) eine zweite Batterie umfasst.

3. Defibrillator nach Anspruch 1, wobei die Stromversorgungsquelle (16) entweder eine Gleichstromquelle oder eine Wechselstromquelle umfasst.

4. Defibrillator nach Anspruch 1, wobei das Leistungssteuerungsmodul (14, 30) eine Steuereinheit (30) umfasst, die konfiguriert ist, um selektiv Verbindungen (32, 34, 36) zwischen der ersten Batterie (12) und der Stromversorgungsquelle (16) und von sowohl der ersten Batterie (12) als auch der Stromversorgungsquelle (16) zum Kondensator (22) herzustellen oder zu trennen.

5. Defibrillator nach Anspruch 1, wobei die Stromversorgungsquelle (16) konfiguriert ist, um die erste Batterie (12) aufzuladen.

6. Defibrillator nach Anspruch 1, weiterhin umfassend eine oder mehrere zusätzliche Batterien, die durch das Leistungssteuerungsmodul gesteuert werden.

7. Defibrillator, umfassend:
eine erste Batterie (12), die konfiguriert ist, um eine Entladungsrate für einen Kondensator bereitzustellen, die für das Erzeugen eines Defibrillierschocks geeignet ist;
eine zweite Batterie (16);
ein Leistungssteuerungsmodul (14), das zwischen die erste Batterie und die zweite Batterie gekoppelt ist und konfiguriert ist, um selektiv Verbindungen zwischen der ersten Batterie und der zweiten Batterie und von sowohl der ersten Batterie als auch von der zweiten Batterie zum Kondensator herzustellen;
eine Abgabeschaltung (20), die mit dem Leistungssteuerungsmodul und dem Kondensator gekoppelt ist und konfiguriert ist, um die Entladung des Kondensators an eine Schockabgabevorrichtung zu ermöglichen, wobei die Abgabeschaltung dem Leistungssteuerungsmodul eine Rückmeldung über den Zustand des Kondensators bereitstellt,
**dadurch gekennzeichnet, dass**
die zweite Batterie (16) eine größere Kapazität hat als die erste Batterie (12), dass das Leistungssteuerungsmodul (14) konfiguriert ist, um basierend auf von der ersten und der zweiten Batterie über die Abgabeschaltung (20) empfangenen Rückmeldungen und auf von dem Kondensator (22) über die Abgabeschaltung (20) empfangenen Rückmeldungen selektiv die genannten Verbindungen herzustellen, um eine Konfiguration von Energiequellen anzupassen, um eine Aufladerate des Kondensators zu erhöhen.

8. Defibrillator nach Anspruch 7, wobei das Leistungssteuerungsmodul (14) eine Steuereinheit (30) umfasst, die konfiguriert ist, um selektiv Verbindungen (32, 34, 36) zwischen der ersten Batterie und der zweiten Batterie und von sowohl der ersten Batterie als auch von der zweiten Batterie zum Kondensator herzustellen oder zu trennen.

9. Defibrillator nach Anspruch 7, wobei die zweite Batterie (16) konfiguriert ist, um die erste Batterie (12) aufzuladen.

10. Defibrillator nach Anspruch 9, weiterhin umfassend eine oder mehrere zusätzliche Batterien, die durch das Leistungssteuerungsmodul gesteuert werden.

11. Verfahren zum Aufladen eines Defibrillators, umfassend:
Bereitstellen (120) einer ersten Batterie, die konfiguriert ist, um eine Entladungsrate für einen Kondensator bereitzustellen, die für das Erzeugen eines Defibrillierschocks geeignet ist, eine Stromversorgungsquelle, und ein Leistungssteuerungsmodul, das mit der ersten Batterie und der Stromversorgungsquelle gekoppelt ist und konfiguriert ist, um selektiv Verbindungen zwischen der ersten Batterie und der Stromversorgungsquelle und von sowohl der ersten Batterie als auch von der Stromversorgungsquelle zum Kondensator herzustellen; und
Aufladen (106) des Kondensators entsprechend dem Leistungssteuerungsmodul, das die Verbindungen entsprechend den Rückmeldungen selektiv herstellt, **dadurch gekennzeichnet, dass**
die Stromversorgungsquelle (16) eine größere Kapazität hat als die erste Batterie (12) hat; dass das Verfahren Folgendes umfasst:
Empfangen (110) von Rückmeldungen von dem Kondensator, der ersten Batterie und der Stromversorgungsquelle beim Leistungssteuerungsmodul (14), um eine Konfiguration von Energiequellen anzupassen, um eine Aufladerate des Kondensators zu erhöhen.

12. Verfahren nach Anspruch 11, wobei die Stromversorgungsquelle eine zweite Batterie umfasst, die konfiguriert ist, um die erste Batterie entsprechend dem Leistungssteuerungsmodul aufzuladen.

13. Verfahren nach Anspruch 11, wobei die Stromversorgungsquelle entweder eine Gleichstromquelle oder eine Wechselstromquelle umfasst.

14. Verfahren nach Anspruch 11, weiterhin umfassend das selektive Herstellen oder Trennen (108) von Verbindungen zwischen der ersten Batterie und der Stromversorgungsquelle und von sowohl der ersten Batterie als auch der Stromversorgungsquelle mit dem Kondensator, um die Aufladerate des Kondensators zu verbessern.

15. Verfahren nach Anspruch 11, weiterhin umfassend mindestens eines von:
(i) Steuern (112) von einer oder mehreren zusätzlichen Batterien durch das Leistungssteuerungsmodul, oder
(ii) Abgeben (114) eines Schocks durch Entladen des Kondensators über eine Schockabgabevorrichtung.

## Revendications

1. Défibrillateur, comprenant :
une première batterie (12) configurée pour fournir un taux de décharge à un condensateur (22) approprié pour générer un choc de défibrillateur ;
une source d'énergie (16) ; et
un module de commande d'énergie (14, 30) accouplé à la première batterie (12) et à la source d'énergie (16) et configuré pour permettre sélectivement des raccordements entre la première batterie (12) et la source d'énergie (16) et, à partir de chacune de la première batterie (12) et de la source d'énergie (16), au condensateur (22), ledit défibrillateur étant **caractérisé en ce que**
la source d'énergie (16) a une capacité plus grande que la première batterie (12), **en ce que**
le module de commande d'énergie (14, 30) est configuré pour permettre sélectivement auxdits raccordements sur la base d'un renvoi d'informations du condensateur (22), provenant de la première batterie (12) et de la source d'énergie (16) d'ajuster une configuration de sources d'énergie pour augmenter un taux de charge du condensateur (22).

2. Défibrillateur selon la revendication 1, dans lequel la source d'énergie (16) inclut une seconde batterie.

3. Défibrillateur selon la revendication 1, dans lequel la source d'énergie (16) inclut l'une d'une source de courant continu et d'une source de courant alternatif.

4. Défibrillateur selon la revendication 1, dans lequel le module de commande d'énergie (14, 30) inclut un dispositif de commande (30) configuré pour permettre ou interdire sélectivement les raccordements (32, 34, 36) entre la première batterie (12) et la source d'énergie (16) et entre chacune de la première batterie (12) et de la source d'énergie (16) au condensateur (22).

5. Défibrillateur selon la revendication 1, dans lequel la source d'énergie (16) est configurée pour charger la première batterie (12).

6. Défibrillateur selon la revendication 1, comprenant en outre une ou plusieurs batteries supplémentaires commandées par le module de commande d'énergie.

7. Défibrillateur, comprenant :
une première batterie (12) configurée pour fournir un taux de décharge à un condensateur approprié pour générer un choc de défibrillateur ;
une seconde batterie (16) ;
un module de commande d'énergie (14) accouplé entre la première batterie et la seconde batterie et configuré pour permettre sélectivement des raccordements entre la première batterie et la seconde batterie et, à partir de chacune de la première batterie et de la seconde batterie, au condensateur ;
un circuit d'administration (20) accouplé au module de commande d'énergie et au condensateur et configuré pour permettre la décharge du condensateur à un dispositif d'administration de choc, le circuit d'administration fournissant un renvoi d'informations au module de commande d'énergie concernant un état du condensateur, **caractérisé en ce que**
la seconde batterie (16) a une capacité plus grande que la première batterie (12), **en ce que**
le module de commande d'énergie (14) est configuré pour permettre sélectivement lesdits raccordements sur la base d'un renvoi d'informations reçues des première et seconde batteries par le biais du circuit d'administration (20) et du renvoi d'informations reçues du condensateur (22) par le biais du circuit d'administration (20) d'ajuster une configuration de sources d'énergie pour accroître un taux de charge du condensateur.

8. Défibrillateur selon la revendication 7, dans lequel le module de commande d'énergie (14) inclut un dispositif de commande (30) configuré pour permettre ou interdire sélectivement les raccordements (32, 34, 36) entre la première batterie et la seconde batterie et entre chacune de la première batterie et de la seconde batterie au condensateur.

9. Défibrillateur selon la revendication 7, dans lequel la seconde batterie (16) est configurée pour charger la première batterie (12).

10. Défibrillateur selon la revendication 9, comprenant en outre une ou plusieurs batteries supplémentaires commandées par le module de commande d'énergie.

11. Procédé de chargement d'un défibrillateur, comprenant :
la fourniture (120) d'une première batterie configurée pour fournir un taux de décharge à un condensateur approprié pour générer un choc de défibrillateur, une source d'énergie, et un module de commande d'énergie accouplé à la première batterie et à la source d'énergie et configuré pour permettre sélectivement des raccordements entre la première batterie et la source d'énergie et, à partir de chacune de la première batterie et de la source d'énergie, au condensateur ; et
le chargement (106) du condensateur en fonction du module de commande d'énergie qui permet sélectivement les raccordements en fonction d'un renvoi d'informations,
**caractérisé en ce que**
la source d'énergie (16) a une capacité plus grande que la première batterie (12), **en ce que** le procédé comprend :
la réception (110) d'un renvoi d'informations du condensateur, de la première batterie et de la source d'énergie au niveau du module de commande d'énergie (14) pour ajuster une configuration de sources d'énergie pour accroître un taux de charge du condensateur.

12. Procédé selon la revendication 11, dans lequel la source d'énergie inclut une seconde batterie configurée pour charger la première batterie en fonction du module de commande d'énergie.

13. Procédé selon la revendication 11, dans lequel la source d'énergie inclut l'une d'une source de courant continu et d'une source de courant alternatif.

14. Procédé selon la revendication 11, comprenant en outre la permission ou l'interdiction sélective (108) des raccordements entre la première batterie et la source d'énergie et entre chacune de la première batterie et de la source d'énergie au condensateur pour améliorer un taux de charge du condensateur.

15. Procédé selon la revendication 11, comprenant en outre au moins l'une de
(i) la commande (112) d'une ou plusieurs batteries supplémentaires par le module de commande d'énergie, ou
(ii) l'administration (114) d'un choc en déchargeant le condensateur par le biais d'un dispositif d'administration de choc.
